(19) Europäisches Patentamt
European Patent Office
Office européen des brevets

(11) Veröffentlichungsnummer : **0 028 758**
**B1**

(12) # EUROPÄISCHE PATENTSCHRIFT

(45) Veröffentlichungstag der Patentschrift : **15.12.82**

(51) Int. Cl.³ : **C 07 C 69/716**, C 07 C 67/343

(21) Anmeldenummer : **80106586.3**

(22) Anmeldetag : **25.10.80**

(54) **Verfahren zur Herstellung von Pivaloylbrenztraubensäurealkylestern und -cycloalkylestern.**

(30) Priorität : **12.11.79 DE 2945657**

(43) Veröffentlichungstag der Anmeldung : **20.05.81 (Patentblatt 81/20)**

(45) Bekanntmachung des Hinweises auf die Patenterteilung : **15.12.82 Patentblatt 82/50**

(84) Benannte Vertragsstaaten : **BE CH DE FR GB IT LI**

(56) Entgegenhaltungen :
**JOURNAL OF THE AMERICAN CHEMICAL SOCIETY, Band 67, Nr. 9, 13. September 1945, Seiten 1 508-1 509 Easton, Penna E.E. ROYALS : « The use of sodium methoxide in the claisen reaction ».**

(73) Patentinhaber : **BAYER AG**
**Zentralbereich Patente, Marken und Lizenzen**
**D-5090 Leverkusen 1, Bayerwerk (DE)**

(72) Erfinder : **Werner, Friedrich, Dr.**
**Petersenstrasse 1**
**D-5000 Köln 91 (DE)**
Erfinder : **Blank, Heinz Ulrich, Dr.**
**Am Geusfelde 35**
**D-5068 Odenthal (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 028 758 B1

# Verfahren zur Herstellung von Pivaloylbrenztraubensäurealkylestern und -cycloalkylestern

Die Erfindung betrifft ein Verfahren zur Herstellung von Pivaloylbrenztraubensäurealkylestern und -cycloalkylestern durch Claisenkondensation von Pinakolin mit Oxalsäureestern.

Aus J. Am. Chem. Soc. *67*, 1508 (1945) ist die Umsetzung von äquimolaren Mengen Pinakolin, Oxalsäurediethylester und Natriummethylat in Methanol bei Raumtemperatur und einer Reaktionszeit von etwa 20 Stunden bekannt, wobei das Reaktionsgemisch zur Aufarbeitung mit Schwefelsäure versetzt wurde und anschließend der Pivaloylbrenztraubensäureethylester mit Benzol extrahiert und nach Abdampfen des Benzols durch Destillation gewonnen wurde. Dieses Verfahren zeigt folgende Nachteile : Die lange Reaktionszeit erlaubt nur eine geringe Raum-Zeit-Ausbeute ; die aufwendige Extraktion und die Verwendung des gesundheitsschädlichen Benzols sind hinderlich für eine technische Durchführung ; es zeigt sich, daß der hierbei erhältliche Methylester der Pivaloylbrenztraubensäure nur in 83 %iger Reinheit anfällt und 15 % des Ethylesters enthält. Eine solche Produktqualität ist für eine Vielzahl von Einsatzmöglichkeiten unbefriedigend. In der angegebenen Zeitschriftenveröffentlichung wird jedoch die Verwendung des Natriummethylats gegenüber dem Natriumethylat betont, weil das erstere besser zu handhaben sei.

Aus Houben-Weyl, Methoden der organischen Chemie, 4. Auflage, Band VIII, Seite 566, 2. Absatz (Georg Thieme Verlag Stuttgart, 1952) ist bekannt, daß die Esterkondensation, wenn einer der Reaktionspartner ein Keton ist, bei möglichst tiefer Temperatur durchgeführt wird, wobei neben anderen Verbindungen die Keton-oxaloester stets unter guter Kühlung dargestellt werden.

Es wurde nun ein Verfahren zur Herstellung von Pivaloylbrenztraubensäureestern der Formel

$$(CH_3)_3C—CO—CH_2—CO—COOR^1 \quad (III),$$

in der

$R^1$ geradkettiges oder verzweigtes $C_1$-$C_{10}$-Alkyl oder gegebenenfalls durch Methyl oder Ethyl substituiertes $C_3$-$C_8$-Cycloalkyl bedeutet,

durch Kondensation von Pinakolin mit Oxalsäuredialkylestern der Formel

$$R^1O—CO—CO—OR^1 \quad (I),$$

in der

$R^1$ die genannte Bedeutung hat,

in Gegenwart von Alkoholaten der Formel

$$(R^2O)_nM \quad (II),$$

in der

$R^2$ geradkettiges oder verzweigtes $C_1$-$C_{10}$-Alkyl oder gegebenenfalls durch Methyl oder Ethyl substituiertes $C_3$-$C_8$-Cycloalkyl bedeutet,

M ein Alkali- oder Erdalkalimetall ist und

n entsprechend der Wertigkeit des Alkali- oder Erdalkalimetalls 1 oder 2 ist,

als Kondensationsmittel gefunden, das dadurch gekennzeichnet ist, daß man die genannten Reaktionskomponenten bei einer Temperatur von − 50 bis + 50 °C vermischt, die Reaktion bei einer Temperatur von 30 bis 200 °C vervollständigt und das Reaktionsgemisch in an sich bekannter Weise mit Mineralsäure behandelt.

Als geradkettiger oder verzweigter Alkylrest mit 1 bis 10 Kohlenstoffatomen seien Methyl, Ethyl, Propyl, Isopropyl, Butyl, Isobutyl, Hexyl, Octyl oder Decyl genannt. Bevorzugtes Alkyl ist solches mit 1 bis 4 Kohlenstoffatomen. Besonders bevorzugt sind Methyl und Ethyl.

Als gegebenenfalls durch Methyl oder Ethyl substituierter Cycloalkylrest mit 3 bis 8 Kohlenstoffatomen seien beispielsweise genannt Cyclopropyl, Cyclobutyl, Cyclopentyl, Cyclohexyl, Cycloheptyl oder Cyclooctyl. Bevorzugtes Cycloalkyl ist Cyclopentyl und Cyclohexyl.

Von den Oxalsäurediestern der Formel (I) werden bevorzugt der Oxalsäuredimethylester und der Oxalsäurediethylester, besonders bevorzugt der Oxalsäuredimethylester, in erfindungsgemäßen Verfahren eingesetzt.

Im erfindungsgemäßen Verfahren können selbstverständlich auch asymmetrische Oxalsäuredialkylester, in denen also die Oxalsäure mit zwei verschiedenen Alkoholen verestert ist, eingesetzt werden, jedoch werden im allgemeinen die symmetrischen Oxalsäuredialkylester eingesetzt.

Alkyl oder Cycloalkyl für $R^2$ kann den gleichen Bedeutungsumfang mit den gleichen Vorzugsbereichen wie für $R^1$ annehmen, wobei $R^1$ und $R^2$ gleich oder verschieden sein können.

Als Alkalimetall sei beispielsweise Lithium, Natrium, Kalium, Rubidium oder Cäsium genannt. Als Erdalkalimetall sei beispielsweise Beryllium, Magnesium, Calcium, Strontrium oder Barium genannt.

Der Index n nimmt bei einem Alkalimetall den Wert 1 und bei einem Erdalkalimetall den Wert 2 an.

Im erfindungsgemäßen Verfahren werden bevorzugt Alkalimetall-Alkoholate, besonders bevorzugt

Natrium-Alkoholate verwendet. In ganz besonders bevorzugter Weise werden Natriummethylat oder Natriumethylat verwendet.

Die Reaktionskomponenten Pinakolin, Oxalsäuredialkylester und Alkoholat werden im allgemeinen in äquivalenten Mengen eingesetzt. Das Alkoholat kann jedoch auch in einem geringen Überschuß, beispielsweise 0,1 bis 15, bevorzugt 5 bis 10, Äquivalentprozente, bezogen auf den eingesetzten Oxalsäuredialkylester, eingesetzt werden. Dieser geringe Überschuß kann dann günstig sein, wenn das eingesetzte Pinakolin oder der eingesetzte Oxalsäuredialkylester einen geringen Wassergehalt aufweist, der durch das überschüssige Alkoholat aufgebraucht wird. Es ist jedoch ebenso möglich, einen Überschuß an einem oder beiden der übrigen Reaktionspartner einzusetzen. Die hat beispielsweise dann Bedeutung, wenn überschüssiges Pinakolin als Lösungsmittel für das erfindungsgemäße Verfahren dient.

Das erfindungsgemäße Verfahren kann grundsätzlich in Abwesenheit oder in Gegenwart eines Lösungsmittels durchgeführt werden. Bei Abwesenheit eines Lösungsmittels nimmt das Reaktionsgemisch im Verlaufe der Reaktion häufig eine breiartige bis feste Konsistenz an. In einem solchen Fall wird das erfindungsgemäße Verfahren beispielsweise vorteilhaft in einem Mahlwerksautoklaven durchgeführt. Wegen der Verfahrenstechnischen Vereinfachung ist jedoch die Durchführung des erfindungsmäßen verfahrens in Gegenwart eines Lösungsmittels bevorzugt.

Als Lösungsmittel für das erfindungsgemäße Verfahren seien außer dem bereits erwähnten überschüssigen Pinakolin solche erwähnt, die unter den Reaktionsbedingungen inert sind, beispielsweise aliphatische, araliphatische oder aromatische Kohlenwasserstoffe, wie Hexan, Heptan, Octan, Petroleumfraktionen, Benzol, Toluol, oder Xylol, aliphatische Alkohole, wie Methanol, Ethanol, Propanol oder Butanol, Ether, wie Diethylether, Tetrahydrofuran oder Dioxan, sowie aromatische Halogenkohlenwasserstoffe, wie Chlorbenzol oder Dichlorbenzol.

Wenn ein Alkohol als Lösungsmittel für das erfindungsgemäße Verfahren eingesetzt wird, so wird bevorzugt der Alkohol eingesetzt, der auch zur Herstellung des Alkoholats verwendet wurde. Beispielsweise ist es möglich, in überschüssigen Alkohol die zur Kondensationsreaktion erforderliche Menge Alkali- oder Erdalkalimetall einzutragen und sodann das Pinakolin und den Oxalsäuredialkylester zu dieser Alkoholat-Lösung zu geben.

In bevorzugter Weise werden jedoch die aliphatischen, araliphatischen oder aromatischen Kohlenwasserstoffe, sowie die halogenierten aromatischen Kohlenwasserstoffe, die mit Wasser nicht mischbar sind, als Lösungsmittel für das erfindungsgemäße Verfahren eingesetzt. Aus dieser Gruppe werden besonders bevorzugt diejenigen eingesetzt, deren Siedepunkt höher ist als der des in der Kondensationsreaktion abgespaltenen Alkohols.

Die Reaktionskomponenten Alkoholat, Oxalsäuredialkylester und Pinakolin können grundsätzlich in jeder beliebigen Reihenfolge in dem bei der bevorzugten Verfahrensvariante benutzten Lösungsmittel zusammengegeben werden. So ist es beispielsweise möglich, das Alkoholat in dem benutzten Lösungsmittel vorzulegen und dann Oxalsäuredialkylester und danach Pinakolin zuzugeben. Es ist jedoch auch möglich, eine Mischung aus Oxalsäuredialkylester und Pinakolin zu dem in dem Lösungsmittel vorgelegten Alkoholat zu geben. Weiterhin ist es möglich, eine Mischung aus Oxalsäuredialkylester und Pinakolin in dem gewünschten Lösungsmittel vorzulegen und dann das Alkoholat, gegebenenfalls gelöst oder suspendiert in einem weiteren Teil des Lösungsmittels, einzutragen.

Die Reaktionskomponenten werden beispielsweise bei einer Temperatur von − 50 bis + 50 °C, bevorzugt von −30 bis + 30 °C, besonders bevorzugt von −10 bis + 20 °C, vermischt. Nach dem Zusammengeben der Reaktionskomponenten wird die Reaktion bei einer Temperatur von 30 bis 200 °C, bevorzugt 35 bis 140 °C, besonders bevorzugt bei 45 bis 120 °C, vervollständigt. In ganz besonders bevorzugter Weise wird die Kondensationsreaktion bei der Siedetemperatur der am niedrigsten siedenden Komponente des Reaktionsgemisches vervollständigt.

Das erfindungsgemäße Verfahren kann grundsätzlich bei Normaldruck, Unterdruck oder Überdruck, bevorzugt bei Normaldruck, durchgeführt werden. Für den Fall, daß die Kondensationsreaktion bei der Siedetemperatur der am niedrigsten siedenden Komponente des Reaktionsgemisches vervollständigt werden soll, kann diese Siedetemperatur durch Anlegen eines Unterdrucks, beispielsweise zwischen 0,1 und 1 bar, oder eines Überdruckes, beispielsweise zwischen 1 und 5 bar, auf den gewünschten Wert eingestellt werden.

In der Kondensationsreaktion entsteht zunächst das Alkali- beziehungsweise Erdalkalisalz des Pivaloylbrenztraubensäureesters. Zur Bildung des freien Pivaloylbrenztraubensäureesters wird das Reaktionsgemisch anschließend in an sich bekannter Weise mit Mineralsäure behandelt.

Die Kondensation und anschließende Säurebehandlung im erfindungsgemäßen Verfahren sei am Beispiel der Umsetzung von Pinakolin, Oxalsäuredimethylester und Natriummethylat durch die folgenden Formelgleichungen erläutert:

$$(CH_3)_3C{-}CO{-}CH_3 + CH_3O{-}CO{-}CO{-}OCH_3 + CH_3ONa$$

$$\longrightarrow (CH_3)_3C{-}CO{-}\underset{\ominus\ \oplus\ Na}{CH}{-}CO{-}COOCH_3 + 2CH_3OH\ ;$$

$$2(CH_3)C{-}CO{-}\underset{\ominus\ \ \overset{\oplus}{Na}}{CH}{-}CO{-}COOCH_3 + H_2SO_4$$

$$\longrightarrow 2(CH_3)_3C{-}CO{-}CH_2{-}CO{-}COOcH_3 + Na_2SO_4$$

Grundsätzlich kann das Reaktionsgemisch, das bei der Kondensationsreaktion anfällt, ohne weitere Vorbehandlung mit Mineralsäure behandelt werden. Als Mineralsäure sei beispielsweise Salzsäure, Salpetersäure, Phosphorsäure und Schwefelsäure, bevorzugt Schwefelsäure, genannt. Die Mineralsäure kann als konzentrierte oder verdünnte Säure angewandt werden. Bevorzugt ist die Anwendung als verdünnte, besonders bevorzugt als verdünnte wäßrige Säure. Der Wassergehalt der wäßrigen Mineralsäure kann in weiten Grenzen schwanken, beispielsweise von 20 bis 99 Gew.-%, bevorzugt 50 bis 90 Gew.-%, bezogen auf die Gesamtmenge an wäßriger Mineralsäure. Die Menge an Mineralsäure wird im allgemeinen so gewählt, daß das als Alkoholat eingesetzte Alkali- oder Erdalkalisalz neutralisiert wird.

Bei der Behandlung mit Mineralsäure fällt ein mehrphasiges Gemisch an, das den freien Pivaloylbrenztraubensäureester, das gebildete Alkali- oder Erdalkalisalz der Mineralsäure in gelöster oder ausgefällter Form je nach Löslichkeit des Salzes und Wassergehalt des Gemisches, den bei der Kondensation entstandenen Alkohol oder, falls $R^1$ und $R^2$ verschieden sind, die entstandenen Alkohole, sowie gegebenenfalls Wasser und gegebenenfalls das bei der Kondensation verwendete Lösungsmittel enthält. Dieses Gemisch kann beispielsweise durch Abfiltrieren ausgefällter Salze oder Extraktion des freien Pivaloylbrenztraubensäureesters, beispielsweise mit Benzol, weiter behandelt werden.

Hierbei treten jedoch Schwierigkeiten durch zum Teil schwer filtrierbare, schmierige Salzniederschläge, sowie durch eine aufwendige Trennung der bei der Extraktion gebildeten verschiedenen Phasen auf.

Es wurde nun gefunden, daß die Schwierigkeiten bei der Behandlung des in der Kondensationsreaktion entstehenden Reaktionsgemisches mit Mineralsäure dadurch aufgehoben werden können, daß man vor der Behandlung mit Mineralsäure die alkoholischen Bestandteile zumindest teilweise aus dem Reaktionsgemisch entfernt. Bevorzugt ist eine weitgehend vollständige Entfernung der alkoholischen Bestandteile.

Dies kann beispielsweise durch einfache Destillation oder durch azeotrope Destillation erfolgen. So kann beispielsweise bei Verwendung eines Alkohols als Lösungsmittel nach Beendigung der Reaktion der Lösungsalkohol und der durch die Kondensation entstandene Alkohol aus dem Reaktionsgemisch abdestilliert werden. In den Fällen, in denen ein nicht-alkoholisches Lösungsmittel verwendet wird, kann jedoch auch der in der Kondensationsreaktion entstehende Alkohol oder, wenn $R^1$ und $R^2$ verschieden sind, die in der Kondensationsreaktion entstehenden Alkohole bereits während der Kondensation aus dem Reaktionsgemisch abdestilliert werden. Hierbei destilliert je nach der Art des verwendeten Lösungsmittels der Alkohol entweder allein aus dem Reaktionsgemisch ab oder als azeotropes Gemisch mit dem verwendeten Lösungsmittel. In einem solchen Fall kann die Temperatur des Reaktionsgemisches von der Siedetemperatur des abgespaltenen Alkohols bis zur Siedetemperatur des verwendeten Lösungsmittels ansteigen, wenn gegen Ende der Kondensationsreaktion kein Alkohol mehr im Reaktionsgemisch vorhanden ist.

Das vom Alkohol oder von den Alkoholen befreite Reaktionsgemisch wird anschließend mit Mineralsäure behandelt. Vor der Behandlung mit Mineralsäure kann sich das Reaktionsgemisch auch gelöst oder suspendiert in einem mit Wasser nicht mischbaren Lösungsmittel befinden. Diese letztere Variante ist bevorzugt. Hierzu kann nach dem Abdestillieren der alkoholischen Bestandteile der Destillationsrückstand in einem mit Wasser nicht mischbaren Lösungsmittel aufgenommen werden. Als mit Wasser nicht mischbare Lösungsmittel können beispielsweise die weiter oben für die Kondensationsreaktion genannten Lösungsmittel mit Ausnahme der dort genannten alkoholischen Lösungsmittel verwendet werden. Bevorzugt werden die dort genannten Kohlenwasserstoffe oder aromatischen Halogenkohlenwasserstoffe verwendet. Selbstverständlich entfällt das Lösen des Reaktionsgemisches in dem mit Wasser nicht mischbaren Lösungsmittel, wenn in der bevorzugten Variante des erfindungsgemäßen Verfahrens bereits ein solches Lösungsmittel für die Kondensationsreaktion verwandt wird und die alkoholischen Bestandteile aus dieser Lösung herausdestilliert werden.

Der Wassergehalt der bevorzugt eingesetzten wäßrigen Mineralsäure soll ausreichend sein, um die bei der Neutralisation entstehenden Salze, bevorzugt die Alkalisalze, besonders bevorzugt die Natriumsalze, zu lösen.

Nach der Behandlung mit Mineralsäure kann die gebildete wäßrige Phase von der gebildeten organischen Phase abgetrennt werden. Der in der organischen Phase enthaltene Pivaloylbrenztraubensäureester kann sodann durch Destillation gewonnen werden.

Wegen der erhöhten Reaktivität des Oxalsäuredimethylesters, verglichen mit den Estern anderer Alkohole, ist dieser Ester auch unter den Kondensationsbedingungen nach dem Stande der Technik bisher für die Kondensation mit Pinakolin nicht eingesetzt worden. Es ist daher eine besondere Ausgestaltung des erfindungsgemäßen Verfahrens, daß zur Bildung des Pivaloylbrenztraubensäuremethylesters Pinakolin mit Oxalsäuredimethylester in Gegenwart von Natriummethylat kondensiert wird. Dies gilt besonders für die Variante, bei der der bei Raumtemperatur feste Oxalsäuredimethylester in

geschmolzener Form aus einer beheizten Vorlage in das Reaktionsgemisch gegeben wird.

Im allgemeinen wird das erfindungsgemäße Verfahren so ausgeführt, daß man das Alkoholat in dem gewählten Lösungsmittel vorlegt, dann den Oxalsäuredialkylester so zugetropft, daß der Ansatz bei der für das Vermischen der Reaktionskomponenten erforderlichen Temperatur gehalten wird, und schließlich das Pinakolin zu diesem Gemisch gibt. Anschließend wird auf die gewählte Reaktionstemperatur aufgeheizt. Die Umsetzung ist in Abhängigkeit von der etwas verschiedenen Reaktivität der Oxalsäuredialkylester mit verschiedenen Alkoholresten, der Höhe der Reaktionstemperatur und von dem gewählten Lösungsmittel manchmal schon innerhalb weniger Minuten beendet. Für den Fall, daß in der bevorzugten Verfahrensvariante als Lösungsmittel für die Kondensation bereits ein mit Wasser nicht mischbares Lösungsmittel gewählt wurde, werden die bei der Kondensationsreaktion entstehenden alkoholischen Bestandteile des Reaktionsgemisches durch Einstellung einer entsprechend hohen Reaktionstemperatur bereits während der Kondensation aus dem Reaktionsgemisch, gegebenenfalls gemeinsam mit einem Anteil des mit Wasser nicht mischbaren Lösungsmittels, herausdestilliert.

Anschließend an die beendete kondensation wird der in einem mit Wasser nicht mischbaren Lösungsmittel gelöste Ansatz mit einer solchen Menge wäßriger Mineralsäure versetzt, die ausreicht, um das Metallion des eingesetzten Alkoholats zu neutralisieren und das dabei entstehende Salz in der wäßrigen Phase aufzulösen. Zur Behandlung des in dem mit Wasser nicht mischbaren Lösungsmittel gelösten Reaktionsgemisches mit der wäßrigen Mineralsäure werden die organische und die wäßrige Phase durch intensives Rühren kräftig vermischt. Nach Beendigung des Rührens trennen sich die wäßrige und die organische Phase einwandfrei. Die wäßrige Phase wird abgelassen, und die organische Phase wird zur Gewinnung des Pivaloylbrenztraubensäureesters durch fraktionierte Destillation aufgearbeitet.

Entsprechend dem Stand der Technik war zu erwarten, daß bei einer Erhöhung der Reaktionstemperatur Zersetzungsreaktion und eine drastische Ausbeuteverminderung eintreten würden. Überraschenderweise wurde gefunden, daß die Kondensation von Pinakolin, Oxalsäuredialkylestern und einem Alkoholat bei erhöhter Temperatur durchführbar ist, wobei verbesserte Ausbeuten und verkürzte Reaktionszeiten und damit eine erheblich günstigere Raum-Zeit-Ausbeute erzielt werden.

Darüber hinaus wird das erfindungsgemäße Verfahren mit Hilfe einfacher, auch in der chemischen Technik leicht durchführbarer Maßnahmen durchgeführt, beispielsweise wird bei der Aufarbeitung die nach dem Stand der Technik erforderliche Extraktion im erfindungsgemäßen Verfahren unnötig.

Die nach dem erfindungsgemäßen Verfahren hergestellten Pivaloylbrenztraubensäureester sind Vorstufen für die Herstellung von Pivaloylessigsäureestern. So kann beispielsweise ein Gemisch von Pivaloylbrenztraubensäuremethylester mit etwa 1/10 seiner Menge an pulverisiertem Glas auf etwa 175 °C erhitzt werden und innerhalb von etwa 5 1/2 Stunden unter Abspaltung von Kohlenmonoxid zum Pivaloylessigsäuremethylester pyrolysiert werden (US-Patent 2.527.306). Die Pivaloylessigsäureester können sodann weiter mit äquimolaren Mengen Anilin oder substituierten Anilinen durch etwa einstündiges Erhitzen in siedendem Xylol zu den gegebenenfalls substituierten α-Pivaloylacetaniliden umgesetzt werden, die Kuppler für gelbe Farben in der Farbphotographie sind (US-Patent 3.265.506).

### Beispiel 1 (zum Vergleich)

Entsprechend der Vorschrift aus J. Am. Chem. Soc. *67,* 1508 (1945) werden 23 g Natrium in 300 ml absolutem Methanol aufgelöst. Zu der durch Kühlung auf Raumtemperatur gehaltenen Natriummethylat-Lösung wird sodann unter Ausschließung von Luftfeuchtigkeit eine Mischung aus 1 Mol Oxalsäurediethylester und 1 Mol Pinakolin im Verlaufe von 1 Stunde zugetropft. Das Gemisch wird 6 weitere Stunden bei Raumtemperatur gerührt und dann über Nacht bei Raumtemperatur stehen gelassen. Dann wird zu dem eisgekühlten Reaktionsansatz eine eiskalte Lösung von 30 g konzentrierter Schwefelsäure in 200 ml Wasser gegeben. Das Gemisch wird 10 Minuten gerührt, in 1 Liter Wasser gegossen und dann mit drei 100 ml-Portionen Benzol extrahiert. Die vereinigten Benzol-Extrakte werden zweimal mit je 100 ml Wasser gewaschen und dann durch Destillation getrennt. Man erhält 127 g Reaktionsprodukt, das nach gaschromatographischer Analyse 83 Gew.-% Pivaloylbrenztraubensäuremethylester und 15 Gew.-% Pivaloylbrenztraubensäureethylester enthält. Die Gehalte an Methyl- bzw. Ethylester in der Menge von 127 g Reaktionsprodukt entsprechen 56,7 % bzw. 9,5 % der theoretischen Ausbeute.

### Beispiel 2

146 g (1 Mol) Oxalsäurediethylester werden bei 5 °C in 367,2 g einer 20 %igen Natriumethylatlösung in Ethanol innerhalb einer Stunde unter Rühren eingetropft. Man läßt 100 g technisches Pinakolin zulaufen und rührt die Mischung weitere 15 Minuten. Dann wird auf 80 °C geheizt und 3 Stunden unter Rückfluß erhitzt. Die Lösung wird anschließend im Rotationsverdampfer bis zur Trokkene eingedampft. Den Rückstand suspendiert man in 200 ml Toluol und rührt ihn mit 253 g wäßriger Schwefelsäure (20,9 gew.-%ig) 1 Stunde bei 50 °C. Die organische Phase wird abgetrennt und destilliert. Man erhält 177 g Pivaloylbrenztraubensäureethylester (88,2 % der theoretischen Ausbeute) mit einer Reinheit von 99,6 % (durch gaschromatographische Analyse).

Beispiel 3

68 g festes Natriumethylat (1 Mol) werden in 300 g Toluol suspendiert. Innerhalb einer Stunde tropft man bei 20 °C 146 g (1 Mol) Oxalsäurediethylester zu und läßt dann 100 g Pinakolin (1 Mol) zulaufen. Man rührt 15 Minuten nach und destilliert dann innerhalb von 3 Stunden über eine 50 cm hohe Füllkörperkolonne 201 g Ethanol-Toluol-Gemisch ab, wobei die Sumpftemperatur von 70 auf 110 °C ansteigt. Der Sumpf wird auf 40 °C gekühlt und mit 234,5 g wäßriger Schwefelsäure (20,9 gew.-%ig) 1 Stunde gerührt. Die Destillation der organischen Phase über eine Vigreuxkolonne liefert bei $Kp_{0,5}$ : 75 °C 176,5 g Pivaloylbrenztraubensäureethylester (87,1 % der theoretischen Ausbeute) mit einer Reinheit von 98,7 % (durch gaschromatographische Analyse).

Beispiel 4

116,6 g Natriummethylat (2,16 Mol) werden in 400 g Toluol suspendiert. Bei 5-20 °C tropft man innerhalb einer Stunde 292 g (2 Mol) Oxalsäurediethylester ein. Dann läßt man rasch 200 g technisches Pinakolin zulaufen. Es wird 15 Minuten nachgerührt ; danach destilliert man innerhalb von 4 Stunden 247 g Methanol-Ethanol-Toluol-Gemisch ab. Die Sumpftemperatur steigt auf 110 °C gegen Ende der Destillation. Der Sumpf wird auf 40 °C gekühlt und mit 506 g wäßriger Schwefelsäure (20,9 gew.-%ig) 1 Stunde gerührt. Phasentrennung mit anschließender Destillation liefert 359 g Destillat, das zu 92,9 % aus Pivaloylbenztraubensäureethylester und zu 6,4 % aus Pivaloylbrenztraubensäuremethylester (89,6 % der theoretischen Ausbeute) besteht.

Beispiel 5

73,4 g (1,08 Mol) Natriumethylat werden in 950 g n-Hexan suspendiert. Innerhalb einer Stunde werden 146 g (1 Mol) Oxalsäurediethylester bei 0-5 °C eingetropft. Man läßt dann 100 g technisches Pinakolin rasch zulaufen. Man heizt auf 60 °C auf und rührt 1 Stunde. Die gaschromatographische Analyse einer mit Schwefelsäure behandelten Probe zeigt, daß die Kondensation beendet ist. An einer 50 cm hohen Füllkörperkolonne destilliert man bei Kp = 62 °C in 30 Stunden zur vollständigen Entfernung des Ethanols 812 g eines Gemisches aus Ethanol und n-Hexan ab. In die Sumpflösung werden bei 40 °C 253 g Schwefelsäure 20,9 %ig eingerührt. Nach Phasentrennung und Destillation erhält man bei $Kp_1$ : 80 °C 180,4 g Pivaloylbrenztraubensäureethylester (89,6 % der theoretischen Ausbeute) mit einer Reinheit von 99,3 % (durch gaschromatographische Analyse).

Beispiel 6

54 g (1 Mol) Natriummethylat werden in 300 g n-Hexan suspendiert. Bei 0-5 °C tropft man innerhalb von 1 Stunde 118 g (1 Mol) auf 60 °C erwärmten Oxalsäuredimethylester ein. Dann werden bei der gleichen Temperatur 100 g Pinakolin rasch zugegeben. Es wird 15 Minuten bei Raumtemperatur gerührt, danach destilliert man innerhalb von 6 Stunden an einer 50 cm hohen Füllkörperkolonne bei $Kp_{760}$ : 50 °C 222 g n-Hexan-Methanol-Gemisch ab. In den Sumpf werden 234,5 g wäßrige Schwefelsäure (20,9 gew.-%ig) eingerührt. Phasentrennung und anschließende Destillation ergeben bei $Kp_8$ : 105 °C 159 g Pivaloylbrenztraubensäuremethylester (85,0 % der theoretischen Ausbeute) mit einer Reinheit von 99,4 % (durch gaschromatographische Analyse).

Beispiel 7

Zu einer Suspension aus 59,4 g (1,1 Mol) Natriummethylat in 200 g auf 5 °C gekühltem Toluol werden innerhalb von 1 Stunde 118 g auf 60 °C erwärmter Oxalsäuredimethylester unter Rühren getropft. Zur Mischung läßt man zügig 100 g (1 Mol) technisches Pinakolin einlaufen. Man rührt 15 Minuten nach und destilliert dann innerhalb von 3 Stunden 90 g Methanol-Toluol-Gemisch über eine Füllkörperkolonne bei $Kp_{760}$ : 67 bis 100 °C ab. Der Sumpf wird auf 40 °C abgekühlt und mit 258 g 20,9 gew.-%iger wäßriger Schwefelsäure versetzt. Nach 1 Stunde gutem Rühren erfolgt Phasentrennung. Destillation bei $Kp_{0,4}$ : 75 °C liefert 163,5 g Pivaloylbrenztraubensäuremethylester (87,0 % der theoretischen Ausbeute) mit einer Reinheit von 99,0 % (durch gaschromatographische Analyse).

Beispiel 8

Bei Zugabe einer auf 50 °C erhitzten Mischung von Pinakolin und Oxalsäuredimethylester wurden bei ansonsten gleicher Arbeitsweise wie in Beispiel 6 eine Menge von 166,7 g Pivaloylbrenztraubensäuremethylester (89 % der theoretischen Ausbeute) erhalten. Reinheit 99,3 %.

Beispiel 9

In 396 g methanolischer Natriummethylatlösung (30 gew.-%ig) werden bei 5 °C 292 g auf 60 °C

erwärmter Oxalsäuredimethylester innerhalb 1 Stunde eingetropft. Dann gibt man zügig 200 g technisches Pinakolin hinzu. Es wird 15 Minuten bei 20 °C und dann 3 Stunden bei Rückflußtemperatur gerührt.

Die Lösung wird am Rotationsverdampfer bis zur Trockene eingeengt, mit 400 g Toluol aufgenommen und bei 50 °C mit 516 g 20,9 gew.-%iger wäßriger Schwefelsäure gerührt. Phasentrennung und Destillation liefern 293 g Pivaloylbrenztraubensäuremethylester (78,6 % der theoretischen Ausbeute). Reinheit 99,8 %.

Beispiel 10

198 g methanolische Natriummethylatlösung (30 gew.-%ig) werden zu 400 g Chlorbenzol gegeben. Innerhalb von 1 Stunde destilliert man bei 65 °C das Methanol ab. Es wird auf 10 °C abgekühlt und innerhalb von 1 Stunde 118 g (1 Mol) geschmolzener Oxalsäuredimethylester eingetragen. Danach läßt man 100 g Pinakolin (1 Mol) zulaufen und rührt 15 Minuten bei Raumtemperatur nach. Es wird aufgeheizt und innerhalb von 2 Stunden das Reaktionsmethanol abdestilliert. Neutralisation mit Schwefelsäure, Phasentrennung und Destillation ergeben 165 g Pivaloylbrenztraubensäuremethylester (87,8 % der theoretischen Ausbeute). Reinheit 99,0 %.

Beispiel 11

Es wird analog Beispiel 9 gearbeitet. Als Lösungsmittel werden 300 g Xylol eingesetzt. Ausbeute : 162,0 g (84,7 % der theoretischen Ausbeute). Reinheit 97,2 %.

Beispiel 12

57,2 g (1,06 Mol) Natriummethylat werden in 400 g entwässertem Pinakolin suspendiert. Zur Suspension tropft man innerhalb von 1 Stunde 118 g geschmolzenen Oxalsäuredimethylester. Dabei wird die Temperatur auf 10 °C gehalten. Man rührt 15 Minuten nach und destilliert dann bei 64 °C 84 g Methanol-Pinakolin-Gemisch ab. Der Sumpf wird auf 50 °C gekühlt und mit 252 g wäßriger Schwefelsäure (20,9 gew.-%ig) versetzt. Phasentrennung und anschließende Destillation ergeben 164,6 g Pivaloylbrenztraubensäuremethylester (87,8 % der theoretischen Ausbeute), Reinheit 99,3 %.

**Ansprüche**

1. Verfahren zur Herstellung von Pivaloylbrenztraubensäureestern der Formel

$$(CH_3)_3C{-}CO{-}CH_2{-}CO{-}COOR^1,$$

in der

$R^1$ geradkettiges oder verzweigtes $C_1$-$C_{10}$-Alkyl oder gegebenenfalls durch Methyl oder Ethyl substituiertes $C_3$-$C_8$-Cycloalkyl bedeutet,

durch Kondensation von Pinakolin mit Oxalsäuredialkylestern der Formel

$$R^1O{-}CO{-}CO{-}OR^1,$$

in der

$R^1$ die genannte Bedeutung hat,

in Gegenwart von Alkoholaten der Formel

$$(R^2O)_nM,$$

in der

$R_2$ geradkettiges oder verzweigtes $C_1$-$C_{10}$-Alkyl oder gegebenenfalls durch Methyl oder Ethyl substituiertes $C_3$-$C_8$-Cycloalkyl bedeutet,

M ein Alkali- oder Erdalkalimetall ist und

n entsprechend der Wertigkeit des Alkali- oder Erdalkalimetalls 1 oder 2 ist,

als Kondensationsmittel, dadurch gekennzeichnet, daß man die genannten Reaktionskomponenten bei einer Temperatur von − 50 bis + 50 °C vermischt, die Reaktion bei einer Temperatur von 30 bis 200 °C vervollständigt und das Reaktionsgemisch in an sich bekannter Weise mit Mineralsäure behandelt.

2. Verfahren nach Anspruch 1, dadurch gekennzeichnet, daß man die Reaktionskomponenten bei einer Temperatur von − 30 bis + 30 °C vermischt und die Reaktion bei einer Temperatur von 35 bis 140 °C vervollständigt.

3. Verfahren nach Anspruch 1 und 2, dadurch gekennzeichnet, daß man die Reaktion in Gegenwart eines Lösungsmittels durchführt.

4. Verfahren nach Anspruch 1 bis 3, dadurch gekennzeichnet, daß man die Reaktion in Gegenwart eines mit Wasser nicht mischbaren organischen Lösungsmittels durchführt.

5. Verfahren nach Anspruch 1 bis 4, dadurch gekennzeichnet, daß man als Lösungsmittel einen aliphatischen, araliphatischen oder aromatischen Kohlenwasserstoff oder einen halogenierten aromatischen Kohlenwasserstoff einsetzt, dessen Siedepunkt höher ist als der des in der Kondensationsreaktion abgespaltene Alkohols.

6. Verfahren nach Anspruch 1 bis 5, dadurch gekennzeichnet, daß man vor der Behandlung mit Mineralsäure die alkoholischen Bestandteile aus dem Reaktionsgemisch entfernt.

7. Verfahren zur Herstellung von Pivaloylbrenztraubensäuremethylester nach Anspruch 1 bis 6, dadurch gekennzeichnet, daß man Pinakolin mit Oxalsäuredimethylester in Gegenwart von Natriummethylat kondensiert.

**Claims**

1. Process for the preparation of pivaloylpyruvic acid esters of the formula

$$(CH_3)_3C—CO—CH_2—CO—COOR^1,$$

in which

$R^1$ denotes straight-chain or branched $C_1$-$C_{10}$ alkyl or $C_3$-$C_8$ cycloalkyl which is optionally substituted by methyl or ethyl,

by the condensation of pinacolin with oxalic acid dialkyl esters of the formula

$$R^1O—CO—CO—OR^1,$$

in which

$R^1$ has the indicated meaning,

in the presence of alcoholates of the formula

$$(R^2O)_nM,$$

in which

$R^2$ denotes straight-chain or branched $C_1$-$C_{10}$ alkyl or $C_3$-$C_8$ cycloalkyl which is optionally substituted by methyl or ethyl,

M is an alkali metal or alkaline earth metal and n is 1 or 2, according to the valency of the alkali metal or alkaline earth metal,

as condensing agents, characterised in that the reactants mentioned are mixed at a temperature of − 50 to + 50 °C, the reaction is brought to completion at a temperature of 30 to 200 °C and the reaction mixture is treated with mineral acid in a manner which is in itself known.

2. Process according to Claim 1, characterised in that the reactants are mixed at a temperature of − 30 to + 30 °C and the reaction is brought to completion at a temperature of 35 to 140 °C.

3. Process according to Claim 1 and 2, characterised in that the reaction is carried out in the presence of a solvent.

4. Process according to Claim 1 to 3, characterised in that the reaction is carried out in the presence of a waterimmiscible organic solvent.

5. Process according to Claim 1 to 4, characterised in that an aliphatic, araliphatic or aromatic hydrocarbon or a halogenated aromatic hydrocarbon, the boiling point of which is higher than that of the alcohol split off in the condensation reaction, is employed as the solvent.

6. Process according to Claim 1 to 5, characterised in that the alcoholic constituents are removed from the reaction mixture before the treatment with mineral acid.

7. Process for the preparation of pivaloylpyruvic acid methyl esters, according to Claim 1 to 6, characterised in that pinacolin is subjected to a condensation reaction with oxalic acid dimethyl ester in the presence of sodium methylate.

**Revendications**

1. Procédé pour la fabrication d'esters d'acide pivaloylpyruvique de formule

$$(CH_3)_3C—CO—CH_2—CO—COOR^1,$$

dans laquelle

$R^1$ représente un groupe alkyle à chaîne droite ou ramifiée en $C_1$-$C_{10}$ ou cycloalkyle en $C_3$-$C_8$ éventuellement substitué par méthyle ou éthyle,

par condensation de la pinacolone avec les esters de dialkyle d'acide oxalique de formule

$$R^1O—CO—CO—OR^1,$$

dans laquelle
$R^1$ a la signification indiquée,
en présence d'alcoolates de formule

$$(R^2O)_nM,$$

dans laquelle
$R^2$ représente un reste alkyle à chaîne droite ou ramifiée en $C_1$-$C_{10}$ ou un reste cycloalkyle en $C_3$-$C_8$ éventuellement substitué par méthyle ou éthyle,
M est un métal alcalin ou alcalino-terreux et
n est égal à 1 ou 2 selon la valence du métal alcalin ou alcalinoterreux,
comme agent de condensation, caractérisé en ce que l'on mélange les composants de réaction mentionnés à une température de − 50 à + 50 °C, on complète la réaction à une température de 30 à 200 °C et on traite le mélange de réaction de manière connue par un acide minéral.

2. Procédé selon la revendication 1, caractérisé en ce que les composants de la réaction ont une température de − 30 à + 30 °C et on complète la réaction à une température de 35 à 140 °C.

3. Procédé selon les revendications 1 et 2, caractérisé en ce que l'on effectue la réaction en présence d'un solvant.

4. Procédé selon les revendications 1 à 3, caractérisé en ce qu'on effectue la réaction en présence d'un solvant organique non miscible avec l'eau.

5. Procédé selon les revendications 1 à 4, caractérisé en ce que l'on utilise comme solvant un hydrocarbure aliphatique, arylaliphatique ou aromatique ou un hydrocarbure aromatique halogéné dont le point d'ébullition est supérieur à celui de l'alcool libéré dans la réaction de condensation.

6. Procédé selon les revendications 1 à 5, caractérisé en ce que l'on élimine les constituants alcooliques du mélange de réaction avant le traitement par l'acide minéral.

7. Procédé pour la fabrication de pivaloylpyruvate de méthyle selon les revendications 1 à 6, caractérisé en ce que l'on condense la pinacolone avec l'oxalate de diméthyle en présence de méthylate de sodium.